(19) Europäisches Patentamt
European Patent Office
Office européen des brevets

(11) Numéro de publication: **0 468 884 B1**

# (12) FASCICULE DE BREVET EUROPEEN

(45) Date de publication du fascicule du brevet: **20.12.95**

(51) Int. Cl.6: **C07D 403/12**, A61K 31/415, A61K 31/41, A61K 31/40

(21) Numéro de dépôt: **91402055.7**

(22) Date de dépôt: **24.07.91**

## (54) Nouveaux dérivés de benzimidazole, leur préparation, et leur application en tant que médicaments

(30) Priorité: **26.07.90 FR 9009563**

(43) Date de publication de la demande:
**29.01.92 Bulletin 92/05**

(45) Mention de la délivrance du brevet:
**20.12.95 Bulletin 95/51**

(84) Etats contractants désignés:
**AT BE CH DE DK FR GB GR IT LI LU NL SE**

(56) Documents cités:
**EP-A- 0 139 993**
**EP-A- 0 297 661**
**US-A- 4 200 641**
**US-A- 4 684 654**

(73) Titulaire: **LABORATORIOS DEL DR. ESTEVE, S.A.**
**Av. Mare de Deu de Montserrat, 221**
**E-08026 Barcelona (ES)**

(72) Inventeur: **Cuberes-Altisent, Maria Rosa**
**Rue Barcelona No.2-D**
**E-08190 Sant Cugat del Valles, Barcelone (ES)**
Inventeur: **Frigola-Constansa, Jordi**
**Av. Diagonal, 299 at.Ia-**
**E-08013 Barcelone (ES)**
Inventeur: **Pares-Corominas, Juan**
**Padilla, 349, 30 3a**
**E-08025 Barcelone (ES)**

(74) Mandataire: **Ahner, Francis et al**
**CABINET REGIMBEAU**
**26, avenue Kléber**
**F-75116 Paris (FR)**

**EP 0 468 884 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention se rapporte à de nouveaux dérivés de benzimidazole, leur procédé de préparation ainsi qu'à leur application en tant que médicaments.

L'état de la technique antérieure le plus proche est illustré par la demande EP-A-0-297 661, décrivant des dérivés de benzimidazole dotés d'une activité anti-histaminique. Les composés antérieurs diffèrent de ceux de la présente invention tant par la nature du substituant en position 1 du noyau benzimidazole que la par la nature du cycle azoté associé.

Les composés objet de la présente invention répondent à la formule générale I

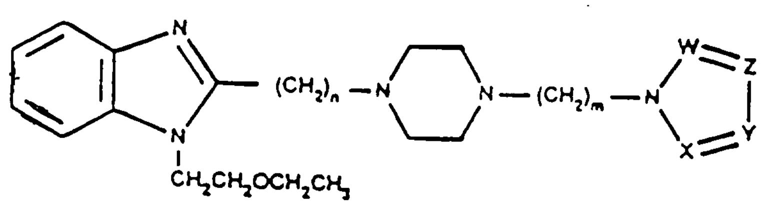

I

dans laquelle :

- n peut avoir les valeurs 0 ou 1,
- m peut avoir les valeurs 2 à 4,
- X, Y, Z et W, égaux ou différents, représentent un atome d'azote ou un atome de carbone lié à un atome d'hydrogène, un halogène ou à un autre radical alkyle, carboxyalkyle, carboxylique, hydroxyle, alkylhydroxy, sulfonique et alkylsulfonique.

Dans la littérature scientifique on connait déjà des dérivés de benzimidazole avec différentes activitées biologiques, comme par exemple, activité analgésique et antiinflamatoire (Japan Kokai 75, 126, 682), activité antisécrétoire gastrique (EP 246.126 et EP 5129); activité antihistaminique (J. Jilek et al., Collect. Czech. Chem. Commun. 1988, 53, 870-83; US Patent 4.200.641; Drugs of the Future, VII; 10-1, 1982; R. Iemura et al., J. Med. Chem., 1986, 29, 1178-1183; R. Iemura et al., J. Heteroxycyclic. Chem., 1987, 24, 31-37). Les composés objet de la présente invention sont des nouveaux dérivés de benzimidazole, en concret 1-(2-étoxyéthyl)-2-(alkylpipérazinylalkylazoles) benzimidazole. Nous avons découvert que ces nouveaux derivés présentent une très bonne activité antihistaminique et ils n'ont pas d' effets secondaires sur le système nerveux central.

Les nouveaux dérivés de formule générale I peuvent être préparés, conformément a l'invention, selon l'un quelconque des méthodes suivantes:

Méthode A .- Par réaction d'un composé de formule générale IIa

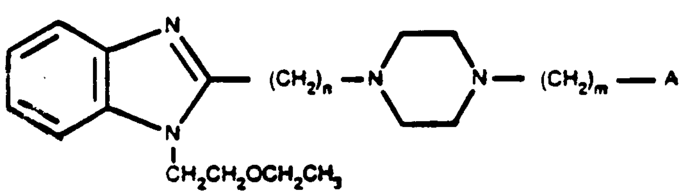

IIa

ou bien IIb

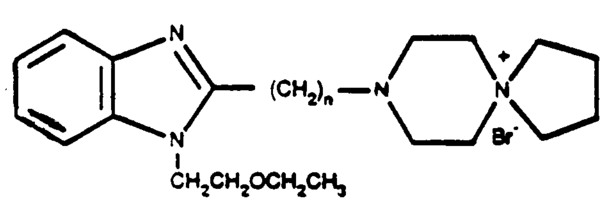

IIb

dans lesquelles n et m ont les significations mentionnées précédemment, et A représente un atome d'halogène, ou un bon "groupe partant" choisi parmi le tosyloxy ou le mésyloxy, avec un composé de formule générale III

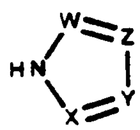

III

dans laquelle X, Y, Z et W ont les significations mentionnées précédemment.

La réaction s'effectue en présence d'un solvant adéquat, par exemple le diméthylsulfoxyde, la diméthylformamide, des alcools, des hydrocarbures, aromatiques ou non, des éthers, tels le dioxanne ou l'éther diphénylique, ou des mélanges de ces solvants. Cette réaction est avantageusement conduite en présence d'une base telle les hydroxydes, les carbonates ou les bicarbonates des métaux alcalins, ou bien d'un mélange de ces bases. On peut employer aussi les hydrures des métaux alcalins. Les températures les plus adéquates oscillent entre la température ambiante et la température de reflux du solvant, et le temps réactionnel est compris entre 1 heure et 24 heures.

Méthode B.- Par réaction d'un composé de formule générale IIa, dans laquelle A représente un radical $-NH_2$, avec le 2,5-diméthoxytétrahydrofurane.

La réaction s'effectue en présence d'un solvant adéquat, par exemple l'acide acétique, de l'eau, des alcools, des cétones ou des mélanges de ces solvants. Les températures les plus adéquates oscillent entre la température ambiante et la température de reflux du solvant, et le temps réactionnel est compris entre quelques minutes et 24 heures.

Méthode C.- Par réaction d'un composé de formule générale IV

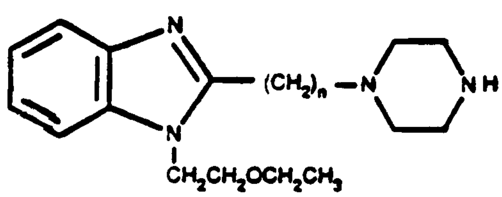

IV

dans laquelle n a la signification mentionnée. précédemment, avec un composé de formule générale V

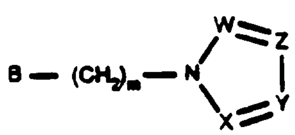

V

où X, Y, Z, W et m ont les significations mentionnées précédemment, et B représente un atome d'halogène, ou un bon "groupe partant" choisi parmi le tosyloxy ou le mésyloxy.

La réaction s'effectue en présence d'un solvant adéquat, par exemple le diméthylsulfoxyde, la diméthylformamide, des alcools, des hydrocarbures, aromatiques ou non, des éthers, tels le dioxanne ou l'éther diphénylique, ou des mélanges de ces solvants. Cette réaction est avantageusement conduite en présence d'une base telle les hydroxydes, les carbonates ou les bicarbonates des métaux alcalins, ou bien d'un mélange de ces bases. Les températures les plus adéquates oscillent entre la température ambiante et la température de reflux du solvant, et le temps réactionnel est compris entre 1 heure et 24 heures.

Dans les exemples suivants on indique la préparation des nouveaux dérivés selon l'invention. Les exemples ci-aprés, donnés à simple titre d'illustration, ne doivent cependant, en aucune façon, limiter l'étendue de l'invention.

Méthode A

Exemple 1.- Préparation de 1-(2-Ethoxyéthyl)-2-{4-[4-(4-bromopyrazol-1-yl)butyl]pipérazin-1-yl-méthyl}benzimidazole.

a) Bromure de 1-(2-Ethoxyéthyl)-2-(8-méthylaza-5-azoniaspiro [4,5] décane)benzimidazole.

On met à reflux pendant 16 heures un mélange de 1,5 g (5,21 mmoles) de 1-(2-Ethoxyéthyl)-2-(1-pipérazinyl)benzimidazole, 1,41 g (6,5 mmoles) de 1,4-dibromobutane et 0,72 g (5,2 mmoles) de carbonate de potassium dans 50 ml de chloroforme. On refroidit, on filtre et on évapore. On triture le résidu dans l'éther éthylique et on obtient 2,1 g de bromure de 1-(2-Ethoxyéthyl)-2-(8-méthylaza-5-azoniaspiro [4,5] décane)benzimidazole, un solide hygroscopique qu'on utilise tel quel, sans autre purification.
$^1$H-RMN ($CDCl_3$) : $\delta$ 1,05 (t,3H); 2,25 (m,4H); 3.25-4,15 (m,18H); 4,45 (t,2H); 7,27 (m,3H); 7,60 (m,1H).

b) 1-(2-Ethoxyéthyl)-2-{4-[4-(4-bromopyrazol-1-yl)butyl] pipérazin-1-yl-methyl}benzimidazole.

On chauffe à reflux, pendant 12 heures, un mélange de 2.3 g (5,44 mmoles) de bromure de 1-(2-Ethoxyéthyl)-2-(8-méthylaza-5-azoniaspiro [4,5] décane)benzimidazole. 0.92 9 (6,28 mmoles) de 4-bromo-1-H-pyrazole, 1,38 g (0,01 moles) de carbonate de potassium et 30 ml de diméthylformamide. On refroidit, on filtre et on évapore le filtrat à sec. On reprend le résidu avec du chloroforme et on lave avec de l'eau. On sèche la phase organique avec $Na_2SO_4$, on filtre et on évapore. L'huile résultant est purifiée sur une colonne chromatographique de sillice (éluant: chloroforme-méthanol 95:5). On obtient ainsi 0,78 g du composé en forme liquide.

Les donnés spectroscopiques pour son identification sont exposées dans les Tableaux 1 et 2.

Exemple 2.- Préparation de 1-(2-Ethoxyéthyl)-2-{4-[4-(4-bromopyrazol-1-yl)butyl]pipérazinyl}benzimidazole.

a) Bromure de 1-(2-Ethoxyéthyl)-2-(8-aza-5-azoniaspiro [4,5] décane)benzimidazole.

La préparation s'effectue avec la même procédure à celle exposée à l'exemple la.
$^1$H-RMN ($CDCl_3$): $\delta$ 1,08 (t,3H); 2,37 (m,4H); 3,42 (q,2H); 3,7-4,15 (m,14H); 4,32 (t,2H); 7,27 (m,3H); 7,60 (m,1H).

b) 1-(2-Ethoxyéthyl)-2-{4-[4-(4-bromopyrazol-1-yl)butyl] pipérazinyl}benzimidazole.

On effectue la préparation avec la même méthode que l'exemple 1b.

Le sel avec l'acide maléique est preparé dans l'éthanol et son point de fusion est 137-139 °C.

Les données spectroscopiques pour son identification sont exposées dans les Tableaux 1 et 2.

Exemple 3.- Préparation de 1-(2-Ethoxyéthyl)-2-{4-[4-(imidazol-1-yl)butyl]pipérazin-1-yl-methyl}benzimidazole.

La préparation s'effectue de manière tout à fait semblable à celle exposée aux exemples 1a et 1b.

Les données spectroscopiques pour son identification sont exposées dans les Tableaux 1 et 2.

Exemple 4.- Préparation de 1-(2-Ethoxyéthyl)-2-{4-[4-(1,2,4-triazol-1-yl)butyl]pipérazin-1-yl-méthyl}benzimidazole.

La préparation s'effectue de manière tout à fait semblable à celle exposée aux exemples 1a et 1b.

Les données spectroscopiques pour son identification sont exposées dans les Tableaux 1 et 2.

Exemple 5.- Préparation de 1-(2-Ethoxyéthyl)-2-{4-[4-(4-sulfopyrazol-1-yl)butyl]pipérazin-1-yl-méthyl}benzimidazole.

La préparation s'effectue de manière tout à fait semblable à celle exposée aux exemples 1a et 1b.

Les données spectroscopiques pour son identification sont exposées dans les Tableaux 1 et 2.

Exemple 6.- Préparation de 1-(2-Ethoxyéthyl)-2-{4-[4-(4-carboxypyrazol-1-yl)butyl]pipérazin-1-yl-méthyl}benzimidazole.

Suivant la préparation exposée à l'exemple 1a et 1b on obtient le 1-(2-Ethoxyéthyl)-2-{4-[4-(4-éthyloxycarbonylpyrazol-1-yl)butyl]pipérazin-1-yl-méthyl}benzimidazole cru, qui est purifié sur une colonne chromatographique de sillice (éluant: chloroforme-méthanol 95:5).
$^1$H-RMN ($CDCl_3$): δ 1,12 (t,3H); 1,45 (m,5H); 1,90 (m,2H); 2,44 (m,10H); 3,41 (q,2H); 3,60-3,86 (m,4H); 4,0-4.3 (m,4H); 4,50 (t,2H); 7,28 (m,3H); 7,75 (m,1H); 7,8 (s,2H).
IR (film): 1715, 1560, 1470, 1225, 1120, 1040, 750 $cm^{-1}$

Le sel avec l'acide maléique est preparé dans l'éthanol et son point de fusion est 114-117 °C.

L'ester précédemment préparé est hydrolysé par traitement d'une solution dans l'éthanol, pendant 3 heures à température ambiante, avec de la soude à 10%. On évapore l'alcool et la solution aqueuse est neutralisée avec de l'acide acétique. On évapore à sec et l'acide est extrait du résidu par digestion avec du dichlorométane.

On obtient ainsi l'acide correspondant dont les données spectroscopiques pour son identification sont exposées dans les Tableaux 1 et 2.

Exemple 7.- Préparation de 1-(2-Ethoxyéthyl)-2-{4-[4-(pyrazol-1-yl)butyl]pipérazin-1-yl-méthyl}benzimidazole.

La préparation s'effectue de manière tout à fait semblable à celle exposée aux exemples 1a et 1b.

Le sel avec l'acide maléique est preparé dans l'éthanol et fond à 112-116 °C.

Les données spectroscopiques pour son identification sont exposées dans les Tableaux 1 et 2.

Exemple 9.- Préparation de 1-(2-Ethoxyéthyl)-2-{4-[4-(4-carboxypyrazol-1-yl)butyl]pipérazin-1-yl}benzimidazole.

Suivant la préparation exposée à l'exemple 2a et 2b on obtient le 1-(2-Ethoxyéthyl)-2-{4-[4-(4-éthyloxycarbonylpyrazol-1-yl)butyl]pipérazin-1-yl}benzimidazole cru, qui est purifié sur une colonne chromatographique de sillice (éluant: chloroforme-méthanol 95:5).
$^1$H-RMN ($CDCl_3$): δ 1,1 (t,3H); 1,3 (t,3H); 1,8 (m,4H); 2,15-2,7 (m,6H); 3,25 (m,6H); 3,7 (t,2H); 3,8-4,3 (m,6H); 7,2 (m,3H); 7,5 (m,1H); 7,8 (s,2H).
IR (KBr): 2950, 1715, 1220, 1125, 760 $cm^{-1}$

L'ester précédemment préparé est hydrolysé par traitement avec de la soude à 10%, pendant 15 heures à température ambiante, d'une solution dans l'éthanol. On évapore l'alcool et la solution aqueuse est neutralisée avec de l'acide chlorhydrique. On évapore à sec et l'acide est extrait du résidu par digestion avec du chloroforme.

On obtient ainsi l'acide correspondant qui est trituré avec l'éther éthylique. Le composé cristallise dans ce solvant avec un point de fusion de 145-150 °C.

Les données spectroscopiques pour son identification sont exposées dans les Tableaux 1 et 2.

Exemple 10.- Préparation de 1-(2-Ethoxyéthyl)-2-{4-[4-(4,5-dichloroimidazol-1-yl)butyl]pipérazin-1-yl-méthyl} benzimidazole.

La préparation s'effectue de manière tout à fait semblable à celle exposée aux exemples 1a et 1b.

Le sel avec l'acide fumarique est preparé dans l'éthanol et fond à 123-130 °C.

Les données spectroscopiques pour son identification sont exposées dans les Tableaux 1 et 2.

Méthode B

Exemple 8.- Préparation de 1-(2-Ethoxyéthyl)-2-{4-[4-(pyrrol-1-yl)butyl]pipérazin-1-yl-méthyl}benzimidazole.

a) 1-(2-Ethoxyéthyl)-2-{4-(4-aminobutyl)pipérazin-1-yl-méthyl}benzimidazole.

On met à reflux pendant 3 heures un mélange de 6,4 g (22 mmoles) de 1-(2-Ethoxyéthyl)-2-(1-méthylpipérazinyl)benzimidazole,6,26 g (22 mmoles) de N-(4-bromobutyl) phtalimide, 4,55 g (33 mmoles) de carbonate de potassium et 4,62 g (30 mmoles) de iodure de sodium dans 100 ml de méthyl éthyl cétone. On réfroidit, on filtre et on évapore le filtrat à sec. On réprend le résidu avec du chloroforme et de

l'eau, on sèche la phase organique avec $Na_2SO_4$, on filtre et on évapore sous vide. L'huile résultant est purifiée sur une colonne chromatographique de sillice (éluant: chloroforme-méthanol 95:5). On obtient ainsi 8.47 g de 1-(2-Ethoxyéthyl)-2-{4-(4-N-phtalimido-butyl)pipérazin-1-yl-méthyl} benzimidazole.

$^1$H-RMN ($CDCl_3$): $\delta$ 1,1 (t,3H); 2,6 (m,4H); 2,45 (m,10H); 3,3 (q,2H); 3,5-3,8 (m,6H); 4,4 (t,2H); 7,15 (m,4H); 7,55 (m,4H).

On chauffe à reflux, pendant 2 heures, une solution de 8,46 g (17,3 mmoles) du composé antérieurement obtenu et 1,73 g (34,6 mmoles) d'hydrate d'hydrazine dans 150 ml d'éthanol. On refroidit, on filtre, on lave avec éthanol et on évapore le filtrat à sec sous vide. On reprend le résidu avec du chloroforme et on lave à l'eau, on sèche, on évapore et on obtient 4,35 g de 1-(2-Ethoxyéthyl)-2-{4-(4-aminobutyl)pipérazin-1-yl-méthyl}benzimidazole, qu'on utilise sans autre purification.

$^1$H-RMN ($CDCl_3$): $\delta$ 1,1 (t,3H); 1,45 (m,4H); 1,75 (s,2H); 2,05-2,75 (m,12H); 3,35 (q,2H); 3,6-3,9 (m,4H); 4,45 (t,2H); 7,20 (m,3H); 7,6 (m,1H).

b) 1-(2-Ethoxyéthyl)-2-{4-[4-(pyrrol-1-yl)butyl]pipérazin-1-yl-mèthyl}benzimidazole.

On chauffe à reflux, pendant 20 minutes une solution de 2 g (5,57 mmoles) de 1-[2-Ethoxyéthyl)-2-{4-(4-aminobutyl)-pipérazin-1-yl-méthyl}benzimidazole et 0,735 g (5,57 mmoles) de 2,5-diméthoxytétrahydrofurane dans 20 ml d'acide acétique. On refroidit, on verse sur l'eau glacée, on neutralise avec $NaHCO_3$ et on extrait avec du chloroforme. On sèche avec $Na_2SO_4$, et on évapore sous vide à sec. On obtient ainsi 2,75 g du composé cru qu'on purifie sur une colonne chromatographique de sillice (éluant: chloroforme-méthanol 94:6).

Le sel avec l'acide fumarique est preparé dans l'éthanol-éther éthylique, avec un point de fusion de 138-142 °C

Les donnés spectroscopiques pour son identification sont exposées dans les Tableaux 1 et 2.

Méthode C

Exemple 6.- Préparation de 1-(2-Ethoxyéthyl)-2-{4-[4-(4-carboxypyrazol-1-yl)butyl]pipérazin-1-yl-méthyl}benzimidazole.

On met à reflux pendant 4 heures un mélange de 5 g (17,36 mmoles) de 1-(2-Ethoxyéthyl)-2-(1-méthylpipérazinyl) benzimidazole, 4,77 g (17,36 mmoles) de 1-(4-bromobutyl)-4-pyrazole carboxylate d'éthyle, 3,60 (26 mmoles) de carbonate de potassium et 3,52 g (23,5 mmoles) de iodure de sodium dans 100 ml de méthyl éthyl cétone. On refroidit, on filtre et on évapore le filtrat à sec. On réprend le résidu avec du chloroforme et de l'eau, on sèche la phase organique avec $Na_2SO_4$, on filtre et on évapore sous vide. Le cru résultant est purifié sur une colonne chromatographique de sillice (éluant: chloroforme-méthanol 95:5) et on obtient ainsi 4,85 g de 1-(2-Ethoxyéthyl)-2-{4-[4-(4-éthyloxycarbonylpyrazol-1-yl)butyl]pipérazin-1-yl-méthyl}benzimidazole.

Les données spectroscopiques du composé sont les mèmes déjà exposées dans l'exemple 6 de la méthode A.

Cet ester est hydrolysé semblablement à la méthode exposée à l'exemple 6 de la méthode A et on obtient l'acide avec les mêmes données spectroscopiques exposées aux Tableaux 1 et 2.

Exemple 8.- Préparation de 1-(2-Ethoxyéthyl)-2-{4-[4-(pyrrol-1-yl)butyl]pipérazin-1-yl-méthyl}benzimidazole.

La préparation s'effectue de manière tout à fait semblable à l'exposée dans l'exemple précedent, et on obtient le composé dont le sel avec l'acide fumarique présente un point de fusion de 137-142 °C.

Les données spectroscopiques pour son identification sont les mêmes exposées aux Tableaux 1 et 2.

Activité pharmacologique

Les produits objet de la présente invention sont des puissants antihistaminiques que se caractérisent par le fait d'être exempts d'effets sédatifs, contrairement à la plupart des antihistaminiques connus.

Activité antihistaminique "in vivo"

L'activité antihistaminique a été étudiée en déterminant la protection face à la mortalité induite par le produit 48/80 chez le rat. Cet essai a été réalisé en suivant la technique décrite par C.J.E. Niemegeers et

cols. (Arch. int. Pharmacodyn., 234, 164-176 (1978). Les produits

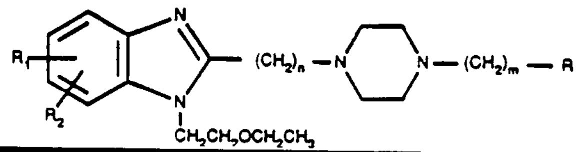

| Exemple nº | $R_1$ | $R_2$ | n | m | R | Méthode | IR ($cm^{-1}$) (film) |
|---|---|---|---|---|---|---|---|
| 1 | H | H | 1 | 4 | 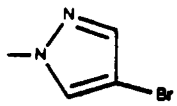 | A | 2937, 2808, 1465, 1117, 952, 747 |
| 2 | H | H | 0 | 4 | 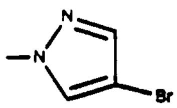 | A | maleate (KBr): 2975, 2881, 1706, 1619, 1475, 1356, 862, 744, 650 |
| 3 | H | H | 1 | 4 | 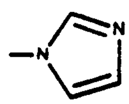 | A | 2938, 2812, 1463, 1132, 749, 665 |
| 4 | H | H | 1 | 4 | 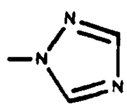 | A | 2940, 2812, 1673, 1463, 1332, 1136, 1011, 749 |
| 5 | H | H | 1 | 4 | 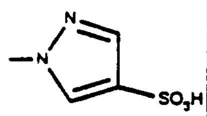 | A | 3600-2800, 1662, 1464, 1220, 1183, 1129, 1052, 670 |
| 6 | H | H | 1 | 4 | 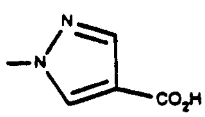 | A<br>C | 3600-3200, 2931, 1706, 1462, 1119, 756 |
| 7 | H | H | 1 | 4 | 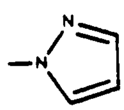 | A | 2838, 2825, 1512, 1462, 1125, 913, 731 |
| 8 | H | H | 1 | 4 | 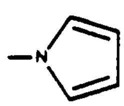 | B<br>C | 2970, 1643, 1463, 1416, 1332, 1120, 749 |
| 9 | H | H | 0 | 4 | 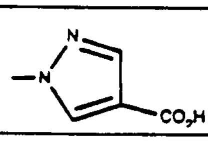 | A | 3360-3150, 2944, 1700, 1525, 1469, 1412, 1125, 750 |
| 10 | H | H | 1 | 4 | 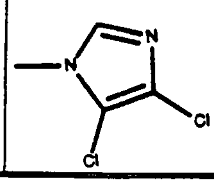 | A | 2940, 2810, 1463, 1254, 749, 666 |

TABLEAU 2

| Exemple nº | $^1H$-RMN ($CDCl_3$) δ |
|---|---|
| 1 | 1,12 (t,3H); 1,46 (m,2H); 1,81 (m,2H); 2,45 (m,10H); 3,41 (q,2H); 3,81 (m,4H); 4,08 (t,2H); 4,50 (t,2H); 7,33 (m,5H); 7,69 (m,1H) |
| 2 | 1,13 (t,3H); 1,60 (m,2H); 1,87 (m,2H); 2,55 (t,2H); 2,76 (m,4H); 3,44 (m,6H); 3,81 (t,2H); 4,12 (dt,4H); 7,12-7,61 (m,6H) |
| 3 | 1,12 (t,3H); 1,70 (m,4H); 2,35 (m,10H); 3,40 (q,2H); 3,75 (m,6H); 4,35 (t,2H); 6,9 (s,1H); 7,0 (s,1H); 7,18-7,45 (m,4H); 7,7 (m,1H) |
| 4 | 1,13 (t,3H); 1,46 (m,2H); 1,90 (m,2H); 2,45 (m,10H); 3,42 (q,2H); 3,76 (t,2H); 3,88 (s,2H); 4,20 (t,2H); 4,52 (t,2H); 7,30 (m,3H); 7,71 m,1H); 7,94 (s,1H); 8,06 (s,1H) |
| 5 | $d_6$-DMSO 1,0(t,3H); 2,11 (m,4H); 3,67 (m,16H); 4,37 (t,2H); 4,65 (t,2H); 7,7 (m,6H) |
| 6 | 1,10 (t,3H); 1,58 (m,2H); 1,86 (m,2H); 2,68 (m,10H); 3,38 (q,2H); 3,73 (t,2H); 3,89 (s,2H); 4,15 (t,2H); 4,48 (t,2H); 7,27 (m,3H); 7,84 (m,3H) |
| 7 | 1,12 (t,3H); 1,50 (m,2H); 1,85 (m,2H); 2,45 (m,10H); 3,41 (q,2H); 3,75 (t,2H); 3,87 (s,2H); 4,14 (t,2H); 4,50 (t,2H); 6,22 (m,1H); 7,19-7,47 (m,6H) |
| 8 | 1,1 (t,3H); 1,70 (m,4H); 2,69 (m,10H); 3,40 (q,2H); 3,55-3,90 (m,6H); 4,46 (t,2H); 6,12 (m,2H); 6,62 (m,2H); 7,1-7,7 (m,4H) |
| 9 | 1,14 (t,3H); 1,59 (m,2H); 1,90 (m,2H); 2,4-2,8 (m,6H); 3,3-3,6 (m,6H); 3,83 (t,2H); 4,17 (dt,4H) |
| 10 | 1,12 (t,3H); 1,55 (m,2H); 1,80 (m,2H); 2,1-2,6 (m,10H); 3,41 (q,2H); 3,86 (m,6H); 4,49 (t,2H); 7,29 (m,4H); 7,75 (m,1H) |

objet de la présente invention s'administrent par voie i.p. aux rats. Après 60 minutes on administre le composé 48/80 (0,5 mg/kg, i.v.). L'activité protectrice se définit comme la survie des rats 4 heures après l'injection i.v. du 48/80.

On étudie l'activité des produits à plusieurs doses à fin de déterminer la dose capable de protéger le 50% des animaux (DE-50).

Ensuite on résume l'activité antihistaminique de quelques uns des produits objet du présent brevet. On compare cette activité avec celle de la difenhidramine, un antihistaminique de référence. La plupart des produits objet de la présente invention sont beaucoup plus actifs que la difenhidramine, étant donné que leur DE-50 est beaucoup plus petite.

Acitivité antihistaminique "in vivo":

| Protection de la mort induite par le 48/80 | |
|---|---|
| Exemple nº | DE-50 (mg/kg, i.p.) |
| 1 | 0.09 |
| 2 | 2.7 |
| 3 | 0.13 |
| 4 | 0.036 |
| 5 | 2.6 |
| 6 | 0.064 |
| 7 | 0.02 |
| 8 | 0.02 |
| 9 | 0.84 |
| 10 | 0.66 |
| Difenhidramine | 5.4 |

Effet sédatif: 1) Test de Irwin

Pour étudier l'abscence d'effet sédatif des produits objet de la présente invention, on les a administré aux rats par voie i.p. et on a observé le comportement des animaux, en suivant les normes décrites dans le test de S Irwin (Science, 136, 123-128 (1962)).

On réunit ci-après les résultats obtenus dans les deux évaluations qui reflètent l'effet sédatif:

- Pas.: Passivité, sédation, prostation. Evaluation quantitative entre 0 et 3. On les réalise 1, 2 et 3 heures après le traitement.
- Atax.: Ataxie, on évalue les altérations de coordination dans la locomotion. On les évalue entre 0 et 3. On les réalise 1, 2 et 3 heures après le traitement.

Ci-après on résume les résultats de l'étude de l'effet sédatif de quelques uns des produits objet de ta présente invention, à titre d'exemple. Cette activité a été comparée avec celle de la difenhidramine, antihistaminique de référence. Les produits objet de la présente invention ont montré un très faible effet sédatif, contrairement à la difenhidramine qui s'est avérée toxique à la dose de 80 mg/kg, i.p., à cause des effets dépresseurs du SNC.

| Effet sédatif: 1) Test de Irwin | | | |
|---|---|---|---|
| Exemple nº | Dose (mg/kg) | Effet | |
| | | Pas. | Atax. |
| 1<br>2 | (80)<br>(40) | 1.4<br>0 | 1.3<br>0.4 |
| <br>3<br>4<br>5 | (80)<br>(80)<br>(80)<br>(80) | 0.7<br>0<br>0.4<br>0.4 | 1.3<br>0<br>0.5<br>0.4 |
| 6<br>7 | (80)<br>(80) | 0<br>0 | 0<br>0 |
| <br>8 | (160)<br>(80) | 0<br>0 | 0.3<br>0 |
| 9<br>Difenhidramine | (80)<br>(40) | 0<br>0 | 0<br>0.9 |
| | (80) | Toxique | |

Effet sédatif: 2) Potentiation du temps de sommeil induit par le pentobarbital

L'étude de la potentiation du temps de sommeil dû au pentobarbital a été réalisée en suivant la méthode décrite par L.E. Allen et cols. (Arz. Forsch. 24, (6), (1974)). Les produits étudiés ont été administrés par voie orale. Une heure plus tard on administre le pentobarbital de sodium (35 mg/kg, s.c.) et on détermine le temps que les animaux tardent à se réveiller. On compare le temps de sommeil avec un groupe d'animaux témoins, traités uniquement avec du pentobarbital de sodium.

Afin de compléter les études qui démontrent l'absence d'effet sédatif des produits objet de la présente invention, on a comparé dans ce test l'activité d'un des produits les plus puissants et avec un moindre effet sédatif (exemple 7) avec l'antihistaminique de référence, la difenhidramine. On présente ci-après les résultats de cet essai avec l'exemple 7 et la difenhidramine. Il est évident que la difenhidramine potentialise de façon significative le temps de sommeil à la dose de 20 mg/kg, alors que l'exemple 7 ne potentialise le temps de sommeil induit par le pentobarbital pas même à 320 mg/kg, dose maximale essayée.

| Effet sédatif : 2) Potentialisation du temps de sommeil induit par le pentobarbital | | |
|---|---|---|
| Exemple nº | Dose (mg/kg, p.o.) | Potentiation temps de sommeil |
| 7 | 80<br>160 | 10 % N.S.<br>11 % N.S. |
| <br>Difenhidramine | 320<br>10 | 22 % N.S.<br>22 % N.S. |
| | 20 | 38 % * |
| N.S.: Non significatif | | |
| * : Différence significative avec le groupe témoin (p < 0,05) | | |

On indiquera ci-après, à titre d'exemple, une forme galénique particulière des dérivés objet de la présente invention.

Comprimés

| Formule par comprimé | |
|---|---|
| Exemple nº 7 | 10,00 mg |
| Lactose | 54,00 mg |
| Amidon de mais | 26,60 mg |
| Cellulose microcristalline | 18,00 mg |
| Polyvinylpyrrolidone | 6,00 mg |
| Croscarmellose de sodium | 3,60 mg |
| Dioxyde sillicique colloïdal | 0,60 mg |
| Stéarate de magnésium | 1,20 mg |
| | 120,00 mg |

**Revendications**

**1.** Composés dérivés de benzimidazole caractérisés en ce qu'ils répondent à la formule générale I, et leurs sels thérapeutiquement acceptables,

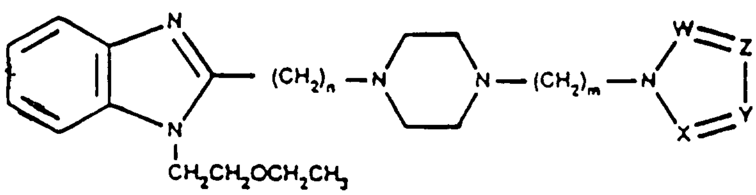

I

dans laquelle :

- n peut avoir les valeurs 0 ou 1,
- m peut avoir les valeurs 2 à 4,
- X, Y, Z et W, égaux ou différents, représentent un atome d'azote ou un atome de carbone lié à un atome d'hydrogène, un halogène ou à un autre radical alkyle, carboxyalkyle, carboxylique, hydroxyle, alkylhydroxy, sulfonique et alkylsulfonique.

**2.** Les composés répondant à la formule générale I selon la revendication 1, sélectionnés parmi le groupe suivant :

- 1-(2-Ethoxyéthyl)-2-{4-[4-(4-bromopyrazol-1-yl)butyl] pipérazin-1-yl-méthyl}benzimidazole.
- 1-(2-Ethoxyéthyl)-2-{4-[4-(4-bromopyrazol-1-yl)butyl] pipérazinyl}benzimidazole.
- 1-(2-Ethoxyéthyl)-2-{4-[4-(imidazol-1-yl)butyl]pipérazin-1-yl-méthyl}benzimidazole.
- 1-(2-Ethoxyéthyl)-2-{4-[4-(1,2,4-triazol-1-yl)butyl] pipérazin-1-yl-méthyl}benzimidazole.
- 1-(2-Ethoxyéthyl)-2-{4-[4-(4-sulfopyrazol-1-yl)butyl] pipérazin-1-yl-méthyl}benzimidazole.
- 1-(2-Ethoxyéthyl)-2-{4-[4-(4-carboxypyrazol-1-yl)butyl] pipérazin-1-yl-méthyl}benzimidazole.
- 1-(2-Ethoxyéthyl)-2-{4-[4-(4-éthyloxycarbonylpyrazol-1-yl)butyl]pipérazin-1-yl-méthyl}benzimidazole.
- 1-(2-Ethoxyéthyl)-2-{4-[4-(pyrazol-1-yl)butyl]pipérazin-1-yl-méthyl}benzimidazole.
- 1-(2-Ethoxyéthyl)-2-{4-[4-(pyrrol-1-yl)butyl]pipérazin-1-yl-méthyl}benzimidazole.
- 1-(2-Ethoxyéthyl)-2-{4-[4-(4-carboxypyrazol-1-yl)butyl] pipérazin-1-yl}benzimidazole.
- 1-(2-Ethoxyéthyl)-2-{4-[4-(4-éthyloxycarbonylpyrazol-1-yl)butyl]pipérazin-1-yl}benzimidazole.
- 1-(2-Ethoxyéthyl)-2-{4-[4-(4,5-dichloroimidazol-1-yl) butyl]pipérazin-1-yl-méthyl}benzimidazole.

**3.** Procédé de préparation des composés selon l'une des revendications 1 et 2, caractérisé par la mise en oeuvre d'au moins l'une des opérations suivantes :

3a- Par réaction d'un composé de formule générale IIa

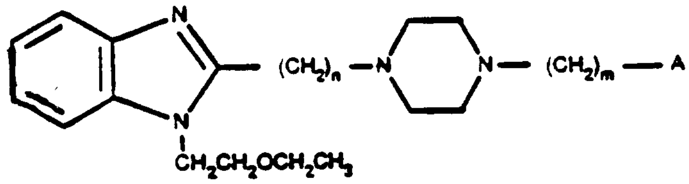

IIa

ou bien IIb

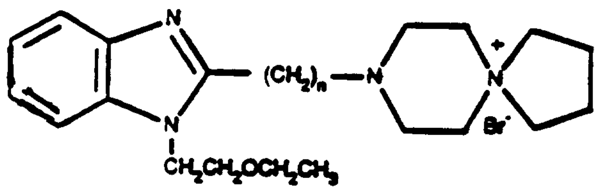

IIb

dans lesquelles n et m ont les significations mentionnées précédemment, et A représente un atome d'halogène, ou un bon "groupe partant" choisi parmi le tosyloxy ou le mésyloxy, avec un composé de formule" générale III

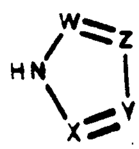

III

dans laquelle X, Y, Z et W ont les significations mentionnées précédemment.

3b- Par réaction d'un composé de formule générale IIa, dans laquelle A représente un radical $-NH_2$, avec le 2,5-diméthoxytétrahydrofurane.

3c- Par réaction d'un composé de formule générale IV

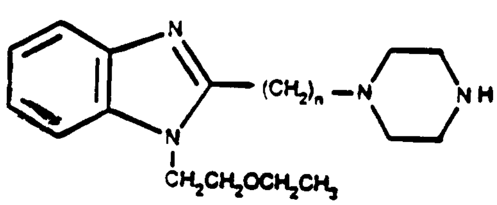

IV

dans laquelle n a la signification mentionnée précédemment, avec un composé de formule générale V

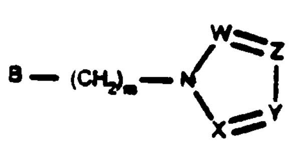

V

où X, Y, Z, W et m ont les significations mentionnées précédemment, et B représente un atome d'halogène, ou un bon "groupe partant" choisi parmi le tosyloxy ou le mésyloxy.

4. A titre de médicaments, les dérivés de formule générale I et leurs sels thérapeutiquement acceptables, selon les revendications 1 et 2, en particulier à titre de médicaments utilisés comme antihistaminiques.

5. Compositions pharmaceutiques, caractérisées par le fait qu'elles contiennent, outre un support pharmaceutiquement acceptable, au moins un dérivé de formule générale I ou l'un de ses sels physiologiquement acceptables, selon l'une des revendications 1 et 2.

6. Utilisation des dérivés de formule générale I et ses sels physiologiquement acceptables, selon l'une des revendications 1 et 2, pour la fabrication de médicaments destinés à la prophylaxie et au traitement de diverses maladies allergiques provoquées par l'histamine.

## Claims

1. Compounds derived from benzimidazole, characterized in that they correspond to the general formula I, and the therapeutically acceptable salts thereof,

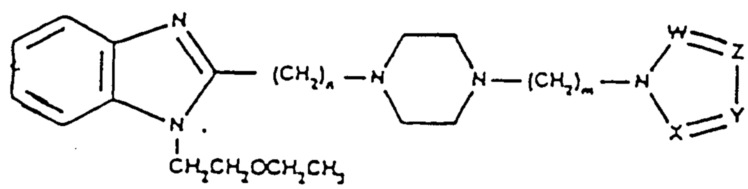

I

in which:
- n may have the values 0 or 1,
- m may have the values 2 to 4,
- X, Y, Z and W, which may be the same or different, represent a nitrogen atom or a carbon atom linked to a hydrogen atom, a halogen or to another alkyl, carboxyalkyl, carboxyl, hydroxyl, alkylhydroxy, sulphonic or alkylsulphonic radical.

2. The compounds corresponding to the general formula I according to Claim 1, which are selected from the following group:
   - 1-(2-ethoxyethyl)-2-{4-[4-(4-bromopyrazol-1-yl)butyl]-piperazin-1-ylmethyl}benzimidazole.
   - 1-(2-ethoxyethyl)-2-{4-[4-(4-bromopyrazol-1-yl)butyl]-piperazinyl}benzimidazole.
   - 1-(2-ethoxyethyl)-2-{4-[4-imidazol-1-yl)butyl]piperazin-1-ylmethyl}benzimidazole.
   - 1-(2-ethoxyethyl)-2-{4-[4-(1,2,4-triazol-1-yl)butyl]-piperazin-1-ylmethyl}benzimidazole.
   - 1-(2-ethoxyethyl)-2-{4-[4-(4-sulphopyrazol-1-yl)butyl]piperazin-1-ylmethyl}benzimidazole.
   - 1-(2-ethoxyethyl)-2-{4-[4-(4-carboxypyrazol-1-yl)butyl]piperazin-1-ylmethyl}benzimidazole.
   - 1-(2-ethoxyethyl)-2-{4-[4-(4-ethyloxycarbonylpyrazol-1-yl)butyl]piperazin-1-ylmethyl}benzimidazole.
   - 1-(2-ethoxyethyl)-2-{4-[4-(pyrazol-1-yl)butyl]piperazin-1-ylmethyl}benzimidazole.
   - 1-(2-ethoxyethyl)-2-{4-[4-(pyrrol-1-yl)butyl]piperazin-1-ylmethyl}benzimidazole.
   - 1-(2-ethoxyethyl)-2-{4-[4-(4-carboxypyrazol-1-yl)butyl]piperazin-1-yl}benzimidazole.
   - 1-(2-ethoxyethyl)-2-{4-[4-(4-ethyloxycarbonylpyrazol-1-yl)butyl]piperazin-1-yl}benzimidazole.
   - 1-(2-ethoxyethyl)-2-{4-[4-(4,5-dichloroimidazol-1-yl)butyl]piperazin-1-ylmethyl}benzimidazole.

3. Process for the preparation of the compounds according to either of Claims 1 and 2, characterized by the use of at least one of the following operations:

3a- By reaction of a compound of general formula IIa

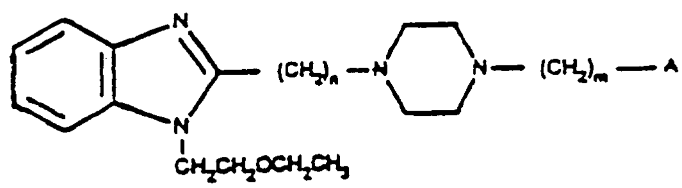

IIa

or alternatively IIb

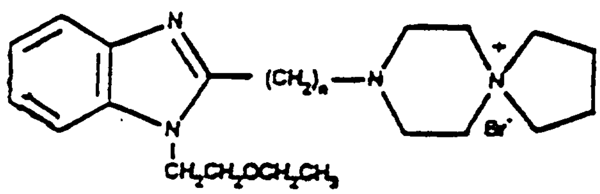

IIb

in which n and m have the meanings mentioned above, and A represents a halogen atom or a good "leaving group" chosen from tosyloxy and mesyloxy, with a compound of general formula III

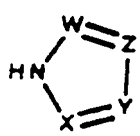

III

in which X, Y, Z and W have the meanings mentioned above.

3b- By reaction of a compound of general formula IIa, in which A represents an $-NH_2$ radical, with 2,5-dimethoxytetrahydrofuran.

3c- By reaction of a compound of general formula IV

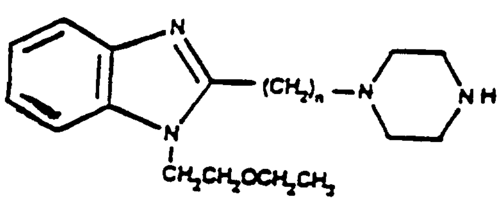

IV

in which n has the meaning mentioned above, with a compound of general formula V

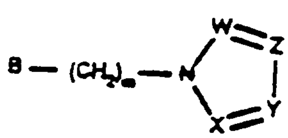

V

where X, Y, Z, W and m have the meanings mentioned above, and B represents a halogen atom or a good "leaving group" chosen from tosyloxy and mesyloxy.

4. By way of medicinal products, the derivatives of general formula I and the therapeutically acceptable salts thereof, according to Claims 1 and 2, in particular as medicinal products used as antihistamines.

5. Pharmaceutical compositions, characterized in that they contain, besides a pharmaceutically acceptable vehicle, at least one derivative of general formula I or one of the physiologically acceptable salts thereof, according to either of Claims 1 and 2.

6. Use of the derivatives of general formula I and the physiologically acceptable salts thereof, according to either of Claims 1 and 2, for the manufacture of medicinal products intended for the prophylaxis and treatment of various allergic diseases caused by histamine.

**Patentansprüche**

1. Benzimidazol-Derivate, dadurch gekennzeichnet, daß sie der allgemeinen Formel (I) entsprechen, und ihre therapeutisch akzeptablen Salze:

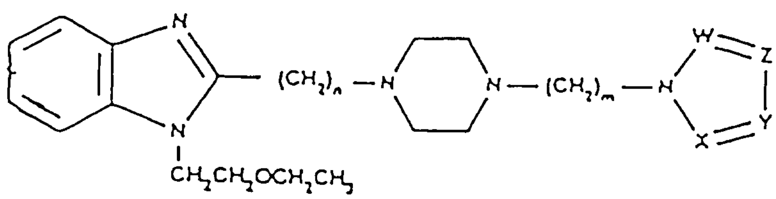

(I)

worin bedeuten:

| | |
|---|---|
| n | die Werte 0 oder 1, |
| m | die Werte 2 bis 4, |
| X, Y, Z und W, | die gleich oder verschieden sind, ein Stickstoffatom oder ein Kohlenstoffatom, das gebunden ist an ein Wasserstoffatom, ein Halogenatom oder einen anderen Alkyl-, Carboxyalkyl-, Carboxyl-, Hydroxyl-, Alkylhydroxy-, Sulfonsäure- und Alkylsulfonsäurerest. |

2. Verbindungen der allgemeinen Formel (I) nach Anspruch 1, ausgewählt aus der folgenden Gruppe:
1-(2-Ethoxyethyl)-2-{4-[4-(4-bromopyrazol-1-yl)butyl]piperazin-1-yl-methyl}benzimidazol;
1-(2-Ethoxyethyl)-2-{4-[4-(4-bromopyrazol-1-yl)butyl]piperazinyl}benzimidazol;
1-(2-Ethoxyethyl)-2-{4-[4-imidazol-1-yl)butyl]piperazin-1-yl-methyl}benzimidazol;
1-(2-Ethoxyethyl)-2-{4-[4-(1,2,4-triazol-1-yl)butyl]piperazin-1-yl-methyl}benzimidazol;
1-(2-Ethoxyethyl)-2-{4-[4-(4-sulfopyrazol-1-yl)butyl]piperazin-1-yl-methyl}benzimidazol;
1-(2-Ethoxyethyl)-2-{4-[4-(4-carboxypyrazol-1-yl)butyl]piperazin-1-yl-methyl}benzimidazol;
1-(2-Ethoxyethyl)-2-{4-[4-(4-ethyloxycarbonylpyrazol-1-yl)butyl]piperazin-1-yl-methyl}benzimidazol;
1-(2-Ethoxyethyl)-2-{4-[4-(pyrazol-1-yl)butyl]piperazin-1-yl-methyl}benzimidazol;
1-(2-Ethoxyethyl)-2-{4-[4-(pyrrol-1-yl)butyl]piperazin-1-yl-methyl}benzimidazol;
1-(2-Ethoxyethyl)-2-{4-[4-(4-carboxypyrazol-1-yl)butyl]piperazin-1-yl}benzimidazol;
1-(2-Ethoxyethyl)-2-{4-[4-(4-ethyloxycarbonylpyrazol-1-yl)butyl]piperazin-1-yl}benzimidazol;
1-(2-Ethoxyethyl)-2-{4-[4-(4,5-dichloroimidazol-1-yl)butyl]piperazin-1-yl-methyl}benzimidazol.

3. Verfahren zur Herstellung der Verbindungen nach einem der Ansprüche 1 und 2, dadurch gekennzeichnet, daß man mindestens eine der folgenden Operationen durchführt:
3a) Umsetzung einer Verbindung der allgemeinen Formel (IIa)

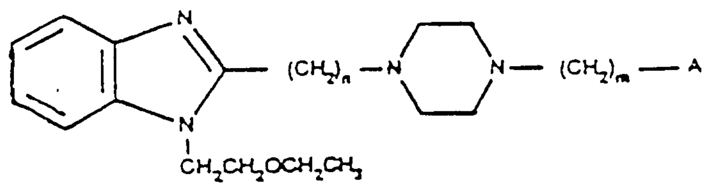

(IIa)

oder auch der allgemeinen Formel (IIb)

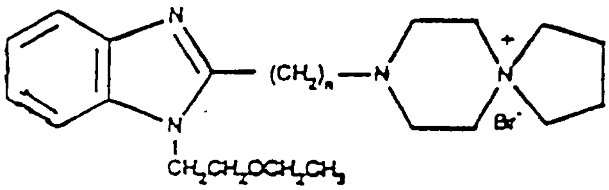

(IIb)

in denen n und m die oben angegebenen Bedeutungen haben und A für ein Halogenatom oder eine leicht "austretende(abspaltbare) Gruppe", ausgewählt aus Tosyloxy oder Mesyloxy, steht, mit einer Verbindung der allgemeinen Formel (III)

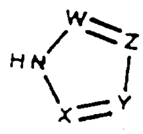

(III)

in der X, Y, Z und W die oben angegebenen Bedeutungen haben;
3b) Umsetzung einer Verbindung der allgemeinen Formel (IIa), in der A einen $NH_2$-Rest darstellt, mit 2,5-Dimethoxytetrahydrofuran;
3c) Umsetzung einer Verbindung der allgemeinen Formel (IV)

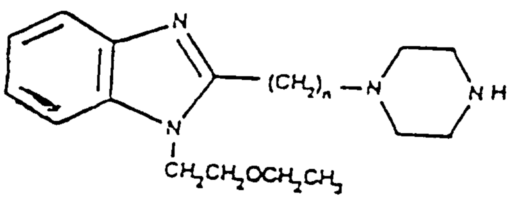

(IV)

in der n die oben angegebene Bedeutung hat, mit einer Verbindung der allgemeinen Formel (V)

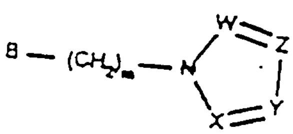

(V)

worin X, Y, Z, W und m die oben angegebenen Bedeutungen haben und B für ein Halogenatom oder eine leicht "austretende(abspaltbare) Gruppe", ausgewählt aus Tosyloxy oder Mesyloxy, steht.

**4.** Derivate der allgemeinen Formel (I) und ihre therapeutisch akzeptablen Salze nach den Ansprüchen 1 und 2 als Arzneimittel, insbesondere als Arzneimittel, die als Antihistaminika verwendet werden.

**5.** Pharmazeutische Zusammensetzungen, dadurch gekennzeichnet, daß sie außer einem pharmazeutisch akzeptablen Träger mindestens ein Derivat der allgemeinen Formel (I) oder eines seiner physiologisch akzeptablen Salze nach einem der Ansprüche 1 und 2 enthalten.

**6.** Verwendung der Derivate der allgemeinen Formel (I) und ihrer physiologisch akzeptablen Salze nach einem der Ansprüche 1 und 2 zur Herstellung von Arzneimitteln für die Prophylaxe und die Behandlung verschiedener allergischer Erkrankungen, die durch Histamin hervorgerufen werden.